# FASCICULE DE BREVET EUROPEEN

(11) **EP 3 152 581 B1**
(45) Date de publication et mention de la délivrance du brevet: **16.10.2019**
(21) Numéro de dépôt: 15732429.4
(22) Date de dépôt: 05.06.2015
(51) Int. Cl.: G01N 33/68

(54) **MARQUEUR BIOLOGIQUE PERMETTANT D'ÉVALUER LE NIVEAU PRO-INFLAMMATOIRE DÉPENDANT DE LA CONTRIBUTION FONCTIONNELLE DE L'IL-17 CHEZ UN INDIVIDU**
BIOLOGISCHER MARKER ZUR BEURTEILUNG DES PROINFLAMMATORISCHEN NIVEAUS IN ABHÄNGIGKEIT VOM FUNKTIONELLEN BEITRAG VON IL-17 BEI EINER PERSON
BIOLOGICAL MARKER FOR EVALUATING THE IL-17 PRO-INFLAMMATORY FUNCTIONAL CONTRIBUTION DEPENDENT LEVEL IN AN INDIVIDUAL

(30) Priorité: 06.06.2014 FR 1455152
(43) Date de publication de la demande: 12.04.2017
(73) Titulaire: Hospices Civils De Lyon, 69002 Lyon (FR); Université Claude Bernard Lyon 1, 69622 Villeurbanne, Cedex (FR)
(72) Inventeur: MIOSSEC, Pierre, F-69500 Bron (FR); THIAM, Ndieme, 38640 Claix (FR)
(74) Mandataire: Nony
(86) Numéro de dépôt international: PCT/IB2015/054268
(87) Numéro de publication internationale: WO 2015/186106

(56) Documents cités:
- FOSSIEZ F ET AL: "T CELL INTERLEUKIN-17 INDUCES STROMAL CELLS TO PRODUCE PROINFLAMMATORY AND HEMATOPOIETIC CYTOKINES", THE JOURNAL OF EXPERIMENTAL MEDICINE, ROCKEFELLER UNIVERSITY PRESS, US, vol. 183, no. 6, 1 juin 1996 (1996-06-01), pages 2593-2603, XP002035506, ISSN: 0022-1007, DOI: 10.1084/JEM.183.6.2593 cité dans la demande
- MARTINE CHABAUD ET AL: "Human interleukin-17: A T cell-derived proinflammatory cytokine produced by the rheumatoid synovium", ARTHRITIS & RHEUMATISM, vol. 42, no. 5, 1 mai 1999 (1999-05-01), pages 963-970, XP055170265, ISSN: 0004-3591, DOI: 10.1002/1529-0131(199905)42:5<963::AID-ANR 15>3.0.CO;2-E cité dans la demande
- PIERRE MIOSSEC ET AL: "Targeting IL-17 and TH17 cells in chronic inflammation", NATURE REVIEWS DRUG DISCOVERY, vol. 11, no. 10, 1 octobre 2012 (2012-10-01), pages 763-776, XP055083929, ISSN: 1474-1776, DOI: 10.1038/nrd3794
- MARDER W ET AL: "Interleukin 17 as a novel predictor of vascular function in rheumatoid arthritis", ANNALS OF THE RHEUMATIC DISEASES, BRITISH MEDICAL ASSOCIATION, LONDON, GB, vol. 70, no. 9, 1 septembre 2011 (2011-09-01), pages 1550-1555, XP008177078, ISSN: 0003-4967, DOI: 10.1136/ARD.2010.148031 [extrait le 2011-07-04]
- NDONGO-THIAM NDIÉMÉ ET AL: "A cell-based bioassay for circulating bioactive IL-17: application to destruction in rheumatoid arthritis", ANNALS OF THE RHEUMATIC DISEASES, B M J GROUP, GB, vol. 74, no. 8, 22 avril 2015 (2015-04-22) , pages 1629-1631, XP008177077, ISSN: 1468-2060, DOI: 10.1136/ANNRHEUMDIS-2014-207110 [extrait le 2015-04-22]
- CHABAUD MARTINE ET AL: "IL-17 derived from juxta-articular bone and synovium contributes to joint degradation in rheumatoid arthritis", ARTHRITIS RESEARCH, CURRENT SCIENCE, LONDON, GB, vol. 3, no. 3, 26 janvier 2001 (2001-01-26), pages 168-177, XP021020631, ISSN: 1465-9905, DOI: 10.1186/AR294
- F. LUZZA ET AL: "Up-Regulation of IL-17 Is Associated with Bioactive IL-8 Expression in Helicobacter pylori-Infected Human Gastric Mucosa", THE JOURNAL OF IMMUNOLOGY, vol. 165, no. 9, 1 novembre 2000 (2000-11-01), pages 5332-5337, XP055170443, ISSN: 0022-1767, DOI: 10.4049/jimmunol.165.9.5332

## Description

### DOMAINE DE L'INVENTION

La présente invention est relative à l'identification d'un nouveau marqueur biologique permettant d'évaluer le niveau pro-inflammatoire dépendant de la contribution fonctionnelle de l'IL-17 chez un individu, à partir d'un échantillon biologique complexe issu dudit individu. L'invention décrit un procédé de détermination du niveau de ce nouveau marqueur, et ses applications diagnostiques et thérapeutiques, tels que définis dans les revendications.

### ART ANTERIEUR

La polyarthrite rhumatoïde est une maladie dégénérative inflammatoire chronique, caractérisée par une atteinte articulaire souvent bilatérale et symétrique, évoluant par poussées vers la déformation et la destruction des articulations atteintes. Le traitement symptomatique fait appel à des anti-inflammatoires non-stéroïdiens et à des corticostéroïdes. De nos jours, le méthotrexate est le traitement de référence. Des traitements « de fond » de la maladie sont activement recherchés, notamment par identification des cytokines impliquées dans le processus inflammatoire de la maladie. Ces traitements font appel à des inhibiteurs spécifiques des cytokines impliquées. On peut citer notamment l'Etanercept® et l'Infliximab®, inhibiteurs du TNF (Tumor Necrosis Factor). D'autres traitements mettent en oeuvre des antagonistes d'interleukines tels que des inhibiteurs de l'IL-1 (Anakinra®), du récepteur de l'IL-6 (MrA®) et les anti-CD20 (Rituximab®). Ces composés réduisent l'inflammation liée au TNF, et ralentissent l'évolution de la maladie.

Néanmoins, une forte variation de réponse est observée entre les patients atteints de polyarthrite rhumatoïde, qui ne répondent pas de manière similaire à ces traitements. On estime notamment que jusqu'à 30 % des patients ne répondent pas du tout aux biothérapies listées ci-dessus.

Il a été montré que l'IL-17 joue un rôle majeur dans les maladies inflammatoires, les maladies auto-immunes et le cancer. Ainsi cette cytokine est maintenant considérée comme une cible thérapeutique potentielle pour de nombreuses maladies (Kolls and Linden, 2004). Les deux formes majoritaires de cette cytokine sont dénommées IL-17A et IL-17F. L'IL-17A induit la production de nombreuses cytokines et chémokines, telles que l'IL-6, le G-CSF, l'IL-1β, et l'IL-8. Dans la polyarthrite rhumatoïde, l'IL-17 est présente aux sites d'inflammation et agit en amplifiant les effets inflammatoires d'autres composés tels que le TNF, ce qui en fait un acteur important dans la physiologie de la maladie. Cependant, les taux circulants d'IL-17 dans le plasma des patients sont souvent très faibles, et une très grande hétérogénéité des taux circulants d'IL-17 est observée entre les patients.

Des inhibiteurs de l'IL-17A, notamment des anticorps, ont été proposés pour le traitement de maladies inflammatoires, et différents essais cliniques sont en cours (Miossec et Kolls, 2012). Les premiers résultats obtenus indiquent qu'il est difficile de prédire quelle sera la réponse des patients à ces traitements, car une très forte variation individuelle existe. Il apparait que chez certains patients, le rôle de l'IL-17 dans le phénomène inflammatoire est important, alors que chez d'autres celui-ci est très faible. Un effort majeur doit être maintenant porté à la pré-sélection des patients dont l'état inflammatoire est directement dépendant de l'IL-17, car ce sont eux qui seraient bénéficiaires de ce type de traitement.

Il est donc important, d'un point de vue clinique, d'identifier un marqueur, autre que le taux circulant d'IL-17 mesuré par ELISA dans le plasma, permettant de déterminer l'importance du rôle fonctionnel de l'IL-17 dans la physiologie et l'évolution de la maladie d'un patient donné. En effet, la simple mesure du taux d'IL-17 circulante dans le plasma d'un patient ne permet pas de déduire l'importance fonctionnelle de l'IL-17 dans le phénomène inflammatoire observé chez ledit patient.

Il a été proposé un procédé pour déterminer le taux d'IL-17 fonctionnelle dans des surnageants de tissus synoviaux, prélevés sur des patients atteints de polyarthrite rhumatoïde ou d'arthrose, puis mis en culture. Ce procédé comprend une étape d'incubation desdits tissus synoviaux sécrétant de l'IL-17, en présence d'anticorps anti-IL-17 (Chabaud *et al.,* 1999). Un tel procédé *in vitro* ne s'apparente pas à une détermination du taux d'IL-17 fonctionnelle dans des échantillons biologiques prélevés sur des patients.

Il n'existe pas, à ce jour, de marqueur permettant de quantifier la part fonctionnelle de l'IL-17 dans un processus inflammatoire à partir d'un échantillon biologique complexe, et donc de déterminer quels sont les maladies et les individus les plus à même d'être traités efficacement par des inhibiteurs d'IL-17.

Par ailleurs, des études ont montré que les patients atteints de maladies inflammatoires chroniques, telles que la polyarthrite rhumatoïde, ont un risque plus élevé de survenue d'un accident cardio-vasculaire que la population générale (Turesson *et al.,* 2007). Or, il n'est pas connu, à ce jour, de test permettant de prédire les patients les plus à risque d'un point de vue cardio-vasculaire, parmi les patients atteints d'un état inflammatoire chronique.

### RESUME DE L'INVENTION

La présente invention décrit un procédé permettant de déterminer, *in vitro,* le niveau pro-inflammatoire dépendant de l'IL-17 fonctionnelle (IPDL pour IL-17 Pro-inflammatory Dependent Level) d'un échantillon biologique issu d'un individu. Ce procédé permet de déterminer, à partir d'un échantillon biologique complexe tel qu'un échantillon de plasma prélevé sur un patient, comprenant d'autres cytokines et notamment du TNF, quelle est la part de l'IL-17 « fonctionnellement active» dans le phénomène inflammatoire observé chez ledit patient. Ce marqueur IPDL permet de mettre en évidence la réelle « bio-disponibilité » de l'IL-17 humaine en présence de nombreuses autres cytokines et facteurs de croissance, optimisant ou inhibant l'activité de l'IL-17, dans un échantillon, lorsque cet échantillon est mis en présence de cellules cibles *in vitro.*

La présente invention concerne donc un procédé *in vitro* pour déterminer le niveau pro-inflammatoire dépendant de l'IL-17 fonctionnelle (IPDL) d'un échantillon biologique choisi parmi du sang total, du plasma et du sérum, comprenant les étapes successives suivantes :
a) mesurer le niveau d'un marqueur inflammatoire produit par des cellules sensibles à l'IL-17, incubées en présence de cet échantillon biologique, lesdites cellules sensibles à l'IL-17 étant des cellules endothéliales,
b) mesurer le niveau dudit marqueur inflammatoire produit par lesdites cellules incubées en présence de ce même échantillon biologique, en présence d'anticorps neutralisant l'activité biologique de l'IL-17, et
c) déterminer la valeur de l'IPDL, qui est la différence entre le niveau dudit marqueur inflammatoire mesuré à l'étape a) et le niveau dudit marqueur inflammatoire mesuré à l'étape b).

La connaissance de ce nouveau marqueur IPDL permet notamment les applications diagnostiques suivantes :
- déterminer les chances de réponse d'un patient atteint d'un état inflammatoire chronique, à un traitement comprenant l'administration d'un anticorps anti-IL17 ;
- déterminer l'efficacité d'un traitement d'un état inflammatoire chronique d'un individu, en particulier lorsque ledit traitement consiste en l'administration d'une composition comprenant un principe actif inhibiteur de l'IL-17 ;
- déterminer le risque de destruction osseuse chez un individu affecté d'un état inflammatoire chronique, notamment de polyarthrite rhumatoïde ; et
- déterminer le risque de survenue d'un accident cardio-vasculaire chez un individu affecté d'un état inflammatoire chronique ; et
- cribler *in vivo* des composés potentiellement inhibiteurs de l'IL-17, chez un animal non humain.

La présente invention est également relative à un kit utile pour déterminer le niveau pro-inflammatoire dépendant de l'IL-17 d'un échantillon choisi parmi du sang total, du plasma et du sérum, prélevé sur un individu, comprenant :
a) un test ELISA destiné à mesurer le taux de sécrétion d'IL-8,
b) un anticorps anti-IL-17, et
c) une lignée immortalisée de cellules endothéliales.

### FIGURES

**Figure 1** **: Mise au point du test destiné à mesurer l'IPDL.**
   Représentation schématique des différentes étapes du procédé décrit. « Patient RA » désigne un patient atteint de polyarthrite rhumatoïde.
**Figure 2** **: Sécrétion d'IL-6 par des synoviocytes/fibroblastes issus de patients atteints de polyarthrite rhumatoïde (RA FLS), stimulés par de l'IL17 recombinante, avec et sans anticorps anti-IL17.**
   Les cellules sont traitées avec de l'interleukine-17 recombinante humaine à 50 ng, avec ou sans anticorps anti-IL17, à des doses de 20, 10, 5 ou 2.5 µg/ml. La production d'IL-6 en ng/ml est mesurée par ELISA.
**Figure 3** **: Détermination de la meilleure concentration de plasma pour le test.**
   Les cellules ont été traitées avec des échantillons de plasmas issus de 8 patients atteints de polyarthrite rhumatoïde, à différentes concentrations (50 %, 20 % et 10 %), le niveau d'IL-6 produite par les cellules a été mesuré par ELISA. Le témoin négatif (barre tout à gauche du graphique) est l'utilisation de milieu de culture DMEM sans ajout de plasma pour l'incubation des cellules.

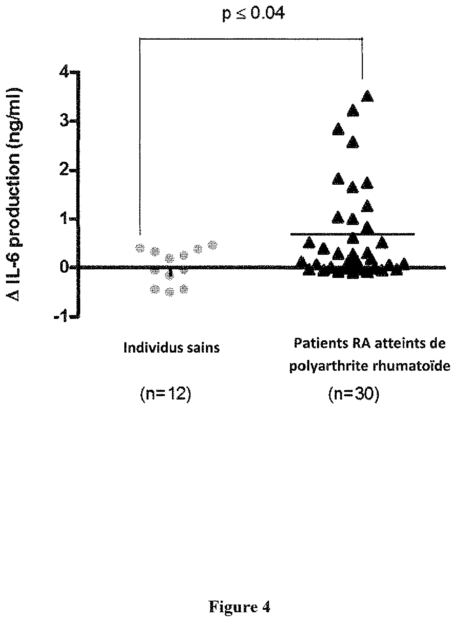

**Figure 4** **: Détermination de l'IPDL chez les patients atteints de polyarthrite rhumatoïde et chez des donneurs sains.**
   10 000 synoviocytes de type fibroblaste (FLS) ont été cultivés dans des plaques à 96 puits dans 200 µl de milieu DMEM, puis les cellules ont été traitées avec un échantillon de plasma de patients à 10 %, avec ou sans anticorps anti-IL17 à 10µg/ml. Les anticorps anti-IL17 et le plasma ont été incubés ensemble pendant 1 h avant l'addition aux cellules FLS. Les surnageants des cellules ont été recueillis et la production d'IL-6 a été mesurée par ELISA. ΔIL-6 (ng/ml) représente la production d'IL-6, avant et après l'addition d'anticorps anti-IL17. Les points témoins sont réalisés avec des cellules traitées avec du plasma (10 % v/v) d'individus en bonne santé. Patients RA plasma (n = 23); Individus sains plasma (n = 11).

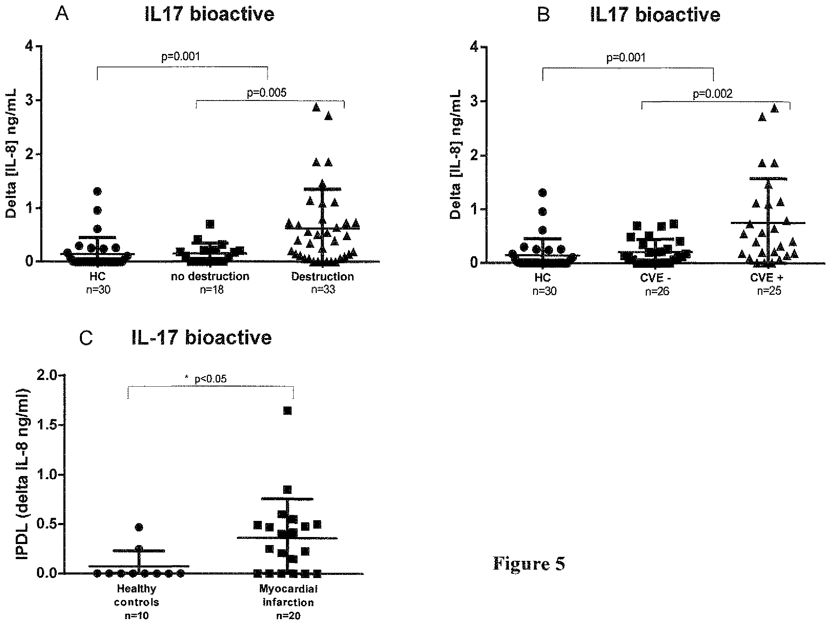

**Figure 5****: Détermination de l'IPDL chez des donneurs sains et des patients atteints de polyarthrite rhumatoïde (A) présentant une destruction osseuse (Destruction) ou non (No Destruction), et (B) ayant connu (CVE+) ou non (CVE-) un accident cardiovasculaire ; (C) : Détermination de l'IPDL chez des patients atteints d'un infarctus du myocarde.**
   10 000 cellules endothéliales (HUVEC) ont été cultivées dans des plaques à 96 puits dans 200 µl de milieu EG-M2, puis les cellules ont été traitées avec 10 % v/v de plasma issus de patients atteints de polyarthrite rhumatoïde (RA) ou d'individus sains (HC) ou de patients en phase aigüe d'un infarctus du myocarde (MI); avec ou sans anticorps anti-IL17 à 10µg/ml. Les anticorps anti-IL17 et le plasma ont été incubés ensemble pendant 4 h avant l'addition aux cellules HUVEC. Les surnageants des cellules ont été recueillis et la production d'IL-8 a été mesurée par ELISA. ΔIL 8 - production (ng / ml) représente la production d'IL-8, avec ou sans addition d'anticorps anti-IL17.
   A- RA no destruction (n=18) ; RA destruction (n=33), HC: Healthy Controls soit individus sains (n=30)
   B- RA plasma CVE - (n = 26), RA plasma CVE + (n = 25), HC : Healthy Controls soit individus sains (n = 30).
   C- HC : Healthy Controls soit individus sains (n = 10) ; MI : patients atteints d'un infarctus du myocarde (n=20).

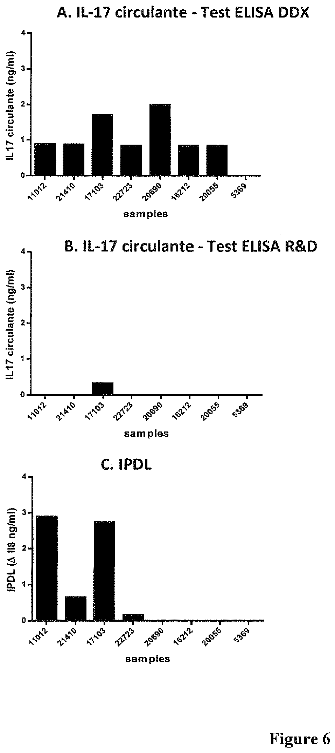

**Figure 6** **: Comparaison du taux d'IL-17 circulante et de l'IPDL dans le plasma de huit patients**
   Dans les échantillons prélevés (samples), le taux d'IL-17 est mesuré grâce à deux tests ELISA (A- ELISA de Dendritics® ; B- ELISA de R&D Systems®). Puis l'IPDL est mesuré sur ces mêmes échantillons par stimulation de cellules endothéliales, et mesure de sécrétion d'IL-8 en présence ou non d'anticorps anti-IL-17 (C).

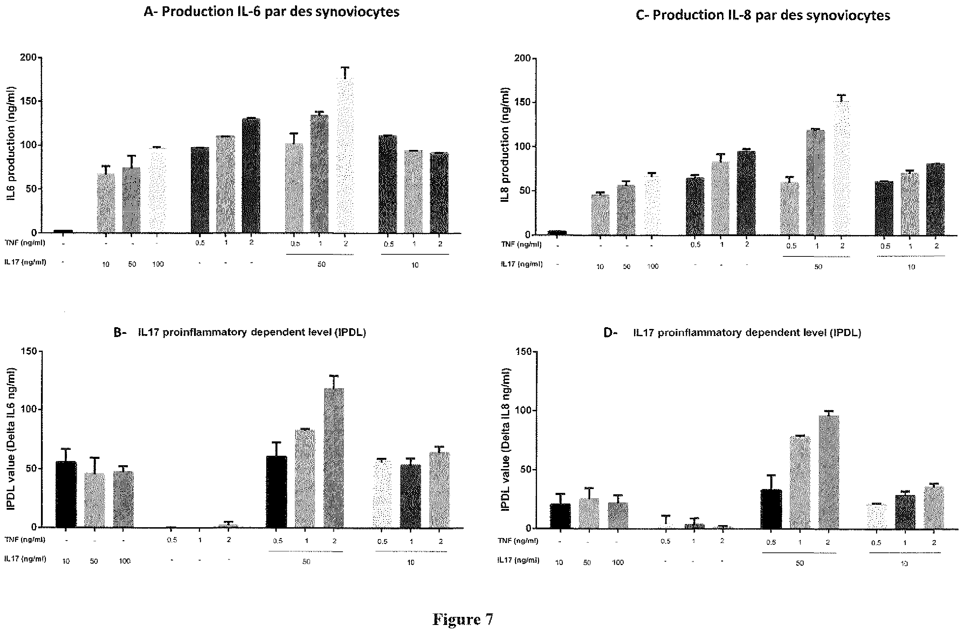

**Figure 7** **: Coopération de l'IL-17 et du TNF dans la stimulation des synoviocytes**
   La production d'Il-6 (A et B) et d'IL-8 (C et D) par des synoviocytes, stimulés par de l'IL-17 recombinante et/ou du TNF recombinant, sont mesurées par ELISA. Puis l'IPDL est calculé sur ces mêmes cellules (B et D), dans les mêmes conditions.

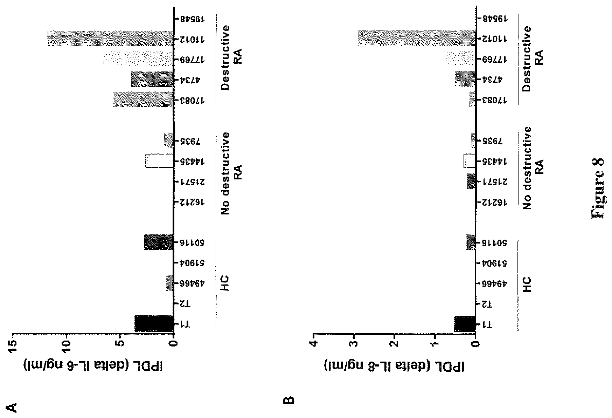

**Figure 8** **: Détermination de l'IPDL par stimulation de cellules endothéliales (A) et de cellules synoviales immortalisées (B).**
   La production d'IL-6 (A) et d'IL-8 (B) par des cellules immortalisées sensibles à l'IL-17, respectivement des cellules FLS immortalisées (A) et des cellules HUVEC (B), stimulées par des échantillons biologiques de patients (16212, 21571, etc) ou de donneurs sains (T1, T2, 49466, etc), selon les conditions adéquates pour déterminer **l'IPDL** desdits échantillons selon le procédé décrit. HC : Healthy Controls soit individus sains; No destructive RA : patients atteints de polyarthrite rhumatoïde, sans destruction osseuse ; Destructive RA : patients atteints de polyarthrite rhumatoïde, avec destruction osseuse.

### DESCRIPTION DETAILLEE DE L'INVENTION

On a montré selon l'invention que la valeur du niveau pro-inflammatoire dépendant de l'IL17 fonctionnelle (IPDL) constituait un nouveau marqueur, indépendant du marqueur « niveau d'IL17 circulante », ce nouveau marqueur étant utile à des fins de diagnostic et de traitement d'états inflammatoires chroniques, notamment de la polyarthrite rhumatoïde. On a montré en particulier que la valeur d'IPDL mesurée dans un échantillon biologique d'un patient permet de déterminer la capacité dudit patient à réagir favorablement à un traitement de l'état inflammatoire chronique avec un inhibiteur de l'IL17, notamment avec un anticorps anti-IL17.

On a aussi montré que la détermination de la valeur du nouveau marqueur IPDL chez un patient affecté d'un état inflammatoire chronique permet d'évaluer le risque d'accident cardio-vasculaire chez ce patient.

On précise que la valeur du nouveau marqueur IPDL mesurée dans un échantillon biologique d'un individu est indépendante d'une valeur spécifique de quantité ou concentration d'IL17 dans cet échantillon, puisque, comme cela est illustré dans les exemples, une valeur donnée d'IPDL, qui est indicative du point de vue diagnostique ou thérapeutique, peut être mesurée dans des échantillons dans lesquels sont mesurées des quantités ou concentrations variées d'IL17.

La présente invention est relative à un procédé *in vitro* pour déterminer le niveau pro-inflammatoire dépendant de l'IL-17 fonctionnelle (IPDL) d'un échantillon biologique choisi parmi du sang total, du plasma et du du sérum, comprenant les étapes suivantes :
a) mesurer le niveau d'un marqueur inflammatoire produit par des cellules sensibles à l'IL-17, incubées en présence dudit échantillon biologique, lesdites cellules sensibles à l'IL-17 étant des cellules endothéliales,
b) mesurer le niveau dudit marqueur inflammatoire produit par lesdites cellules incubées en présence dudit échantillon biologique, en présence d'anticorps neutralisant l'activité biologique de l'IL-17, et
c) déterminer la valeur de l'IPDL, qui est la différence entre le niveau dudit marqueur inflammatoire mesuré à l'étape a) et le niveau dudit marqueur inflammatoire mesuré à l'étape b).

La présente invention se propose de déterminer la contribution fonctionnelle de l'IL-17 dans un échantillon biologique de composition complexe, comprenant de multiples facteurs interagissant entre eux. En effet, dans un tel échantillon, sont présents des facteurs qui interagissent avec l'IL-17 de manière positive, telles que les cytokines TNF et IL-1 par exemple, et des facteurs interagissant avec l'IL-17 de manière négative, inhibant l'action de l'IL-17, notamment comme la cytokine IL-25.

La présente invention propose un procédé pour déterminer la contribution fonctionnelle de l'IL-17 au sein d'un organisme, en se plaçant au plus près des conditions physiologiques dudit organisme, en particulier en présence des cytokines citées ci-dessus, ce qui permet de déterminer le rôle clinique de l'IL-17, et ainsi d'évaluer les chances de réponse de l'organisme d'un patient à un traitement basé sur l'inhibition de l'IL-17.

L'échantillon biologique est prélevé sur un individu, notamment un patient atteint d'un état inflammatoire chronique, et plus particulièrement un patient atteint de l'un des troubles cités dans la présente demande.

L'activité biologique de l'IL-17 recombinante est mesurée en fonction de sa capacité à induire la sécrétion d'un marqueur inflammatoire par des cellules sensibles à l'IL-17, en particulier la sécrétion de certaines interleukines par des fibroblastes ; cet effet est complètement supprimé en présence d'anticorps anti-IL-17, ce qui prouve le rôle essentiel de l'IL-17 (Fossiez *et al.,* 1996). Ce test a été mis au point avec une protéine IL-17 recombinante pure, et non un échantillon biologique complexe, comprenant de l'IL-17 en présence de nombreux autres facteurs, potentiellement modulateurs de la « bio-disponibilité » de l'IL-17.

L'IL-17 ou IL17 désigne l'interleukine-17 telle qu'identifiée en 1993 par l'équipe de Rouvier *et al.* Elle a été re-baptisée en IL-17A après l'identification de nouveaux membres de la famille identifiés, dénommées IL-17B à IL-17F. L'IL-17 est une glycoprotéine homodimérique de 155 acides aminés, d'un poids de 35 kDa. L'IL-17 est sécrétée par des lymphocytes CD4+ et CD8+ et est impliquée dans la coordination de l'inflammation locale des tissus, notamment via l'induction de sécrétion de cytokines pro-inflammatoires et induisant la mobilisation des neutrophiles (Kolls and Linden, 2004).

La présente invention concerne tout particulièrement l'IL-17A, mais peut également être appliquée à d'autres membres de la famille : IL-17B, IL-17C, IL-17D, IL-17E et en particulier IL-17F qui présente le plus fort taux d'identité de séquence avec l'IL-17A, sous réserve que des anticorps spécifiquement dirigés contre ces protéines soient disponibles, et que le test soit pertinent d'un point de vue clinique.

Le terme « Anticorps neutralisant l'activité biologique de l'IL-17 » désigne un anticorps se liant à l'IL-17 de manière à neutraliser son action biologique. Un tel anticorps possède la capacité de bloquer la propriété de l'IL-17 à induire la production de cytokines, comme l'IL-6 et l'IL-8, par des cellules sensibles à l'IL-17 comme les cellules mésenchymateuses ou des cellules endothéliales. Dans certains cas, un tel anticorps bloque l'activité de l'IL-17 en empêchant la liaison de l'IL-17 à son récepteur sur lesdites cellules sensibles. L'invention met en oeuvre en particulier un anticorps neutralisant dirigé contre l'interleukine 17A humaine.

Selon un aspect particulier des procédés selon l'invention, le marqueur inflammatoire est avantageusement choisi parmi l'IL-6, l'IL-8, le G-CSF, le GM-CSF et la prostaglandine E2, et de manière tout à fait préférée parmi l'IL-6 et l'IL-8.

En effet, l'IL-17 induit la sécrétion de facteurs pro-inflammatoires par ses nombreuses cellules cibles, chaque type de cellules cibles étant spécialisé dans la production et sécrétion d'une ou plusieurs cytokines ou chemokines.

L'expression « cellules sensibles à l'Il-17 » ou « cellules cibles » désigne des cellules exprimant le récepteur de l'IL-17, notamment de l'IL-17A, et possédant la voie de signalisation adéquate permettant de transmettre le signal généré par la fixation de l'IL-17 à son récepteur. L'homme du métier sait choisir parmi plusieurs types cellulaires ceux qui seront le plus adaptés pour mettre en oeuvre le procédé selon l'invention.

Les cellules sont incubées, c'est-à-dire mises en contact selon un protocole classique connu de l'homme du métier, en présence d'un échantillon biologique, optionnellement dilué dans du milieu d'incubation approprié pour les cellules.

Selon un aspect particulier décrit, les cellules sensibles à l'IL-17 sont choisies parmi :
- des cellules mésenchymateuses telles que les synoviocytes, les fibroblastes de peau, et les cellules épithéliales, et
- des cellules endothéliales.

Les cellules mésenchymateuses sont issues des cellules souches présentes dans le mésenchyme de l'embryon, qui se différencient en de nombreux types cellulaires. Les cellules endothéliales sont constitutives de l'endothélium, leur rôle principal est de constituer la paroi des vaisseaux sanguins.

Selon un aspect particulier décrit, les cellules cibles sont des cellules de type fibroblastes qui sécrètent de l'interleukine-6 en réponse à la stimulation par l'IL-17.

Le terme « synoviocytes » désigne des cellules de la membrane synoviale, qui produisent le liquide synovial. Elles comprennent notamment les cellules « Fibroblast-Like-Synoviocytes » (FLS).

Selon un aspect de l'invention, les cellules cibles sont des cellules endothéliales qui sécrètent de l'interleukine-8 en réponse à la stimulation par l'IL-17.

Selon un aspect particulier de l'invention, l'échantillon biologique sur lequel est réalisée la mesure est du plasma sanguin, appelé communément 'plasma', c'est-à-dire du sang dont les cellules (notamment les hématies) ont été éliminées par centrifugation après prélèvement sanguin. Le plasma est la partie liquide du sang dans laquelle baignent les composants majeurs tels que les nutriments, cytokines et facteurs de croissance. Cet échantillon biologique est particulièrement complexe du fait de la présence de nombreux composés actifs, présentant des interactions multiples entre eux.

Selon un aspect préféré de l'invention, le plasma n'est pas purifié, et l'échantillon prélevé est déposé tel quel sur les cellules sensibles à l'IL-17 dans le test.

Selon un aspect particulier de l'invention, le plasma est décomplémenté, c'est-à-dire chauffé de manière à inhiber le complément qui peut gêner les réactions antigène-anticorps ; de préférence, le plasma sera traité à 56°C pendant 30 minutes.

Le plasma sanguin est utilisé dans le procédé selon l'invention pour stimuler la sécrétion de marqueur inflammatoire par des cellules sensibles à l'interleukine-17. Le plasma peut être ajouté au milieu de culture de ces cellules selon plusieurs modes, et notamment à différentes concentrations. Le plasma peut contenir de nombreuses cytokines (ILI7, TNF, IL1...) qui sont toutes capables d'induire des niveaux élevés de sécrétion d'IL-6 par des fibroblastes primaires en culture. L'utilisation d'un anticorps anti-IL-17 permet de déterminer quelle est « la part » ou « la contribution » de l'IL-17 dans cette activation.

De manière préférée, les cellules sensibles à l'IL-17 sont incubées, à chacune des étapes a) et b), avec une solution comprenant 10 % volume/volume (v/v) de plasma, soit une préparation contenant 90 % de milieu de culture ou de tampon d'incubation, et 10 % de plasma.

Dans le procédé selon l'invention, la mesure du niveau du marqueur inflammatoire sécrété par les cellules sensibles à l'IL-17 effectuée aux étapes a) et b), peut être réalisée par tout type de test adapté, et déterminable facilement par l'homme du métier. De manière préférée, ledit test sera un test immunologique. Selon un aspect de l'invention, le marqueur (IL-6, IL-8 ou autre selon le type de cellules) sera de préférence mesuré grâce à un test immuno-enzymatique. Ledit test sera notamment un test ELISA, bien connu de l'homme du métier, permettant de mesurer le taux de sécrétion d'IL-6 ou d'IL-8 ou d'un autre marqueur grâce à des anticorps adaptés.

Selon un aspect préféré de l'invention, l'anticorps neutralisant l'activité biologique de l'IL-17 est un anticorps monoclonal. En particulier il s'agira d'un anticorps monoclonal dirigé contre l'Il-17A humaine. Cet anticorps pourra notamment être choisi parmi des anticorps commerciaux. En particulier l'anticorps MAB317 commercialisé par R&D Systems® est indiqué pour la mise en oeuvre de l'invention.

### Applications du procédé de l'invention

La présente invention est également relative à un procédé *in vitro* pour déterminer les chances de réponse d'un patient atteint d'un état inflammatoire chronique, à un traitement comprenant l'administration d'un anticorps anti-IL17, comprenant les étapes suivantes :
a) déterminer la valeur de IPDL d'un échantillon biologique dudit individu, par le procédé tel que décrit ci-dessus, et
b) comparer la valeur de IPDL obtenue à l'étape a) avec une valeur de référence.

On entend par l'expression « état inflammatoire chronique » un état de santé d'un individu, où ledit individu présente à un ou plusieurs endroits du corps une réaction inflammatoire, et notamment la sécrétion de cytokines pro-inflammatoires, et ce de manière continue sur une longue période de temps.

On entend par « chances de réponse » la probabilité qu'un traitement comprenant l'administration d'un inhibiteur de l'IL-17 au patient, en particulier l'administration d'un anticorps anti-IL17 au patient, soit efficace, c'est-à-dire provoque une diminution ou bloque l'état inflammatoire chronique chez ledit patient.

On entend par « échantillon biologique dudit individu » un échantillon de liquide biologique prélevé sur ledit individu, choisi parmi du sang total, du plasma et du sérum. De manière préférentielle, cet échantillon est du plasma sanguin.

Aux fins de réaliser le procédé ci-dessus, on entend par « valeur de référence », une valeur IPDL mesurée chez des patients affecté d'un état inflammatoire chronique, par exemple une polyarthrite rhumatoïde, et dont la réponse à l'administration d'un inhibiteur de IL-17, en particulier l'administration d'un anticorps anti-IL17, est connue. En général, ladite valeur de référence est une valeur IPDL moyenne mesurée à partir d'un échantillon biologique d'une pluralité d'individus affectés d'un état inflammatoire chronique, par exemple d'une polyarthrite rhumatoïde, et dont la capacité à répondre à l'administration d'un inhibiteur de l'IL17, par exemple la capacité à répondre à l'administration d'un anticorps anti-IL17, est connue.

Aux fins de mise en oeuvre des procédés selon l'invention, on peut choisir comme « valeur de référence » une valeur d'IPDL moyenne déterminée ou mesurée chez des patients affectés d'un état inflammatoire chronique, par exemple d'une polyarthrite rhumatoïde, et dont la capacité à répondre à un traitement par un inhibiteur de IL-17, en particulier à un traitement par un anticorps anti-IL-17, est connue.

Dans certains modes de réalisation du procédé, la valeur de référence est une valeur d'IPDL déterminée chez des patients « non-répondeurs », c'est-à-dire chez des patients chez lesquels l'administration d'un inhibiteur de l'IL17, par exemple un anticorps anti-IL17, de provoque pas de diminution d'un état inflammatoire chronique, par exemple de diminution de la polyarthrite rhumatoïde. Dans ces modes de réalisation, un patient testé selon le procédé de l'invention sera classé « non-répondeur » lorsque la valeur d'IPDL mesurée pour ce patient est égale ou inférieure à la valeur IPDL de référence.

Dans certains modes de réalisation du procédé, la valeur de référence est une valeur d'IPDL déterminée chez des patients « répondeurs », c'est-à-dire chez des patients chez lesquels l'administration d'un inhibiteur de l'IL17, par exemple un anticorps anti-IL17, provoque une diminution d'un état inflammatoire chronique, par exemple une diminution de la polyarthrite rhumatoïde. Dans ces modes de réalisation, un patient testé selon le procédé de l'invention sera classé « répondeur » lorsque la valeur d'IPDL mesurée pour ce patient est égale ou supérieure à la valeur IPDL de référence.

Dans encore d'autres modes de réalisation, la valeur de référence est une valeur IPDL dite « seuil » ou de « cut-off », qui est déterminée à partir de valeurs IPDL déterminées chez des patients « répondeurs » et de valeurs IPDL déterminées chez des patients « non-répondeurs ». Dans ces modes de réalisation, un patient testé selon le procédé de l'invention sera classé « non-répondeur » lorsque la valeur d'IPDL mesurée pour ce patient est inférieure à la valeur IPDL de référence Dans ces modes de réalisation, un patient testé selon le procédé de l'invention sera classé « répondeur » lorsque la valeur d'IPDL mesurée pour ce patient est supérieure à la valeur IPDL de référence.

Une valeur de référence d'IPDL « seuil » ou de « cut-off » peut être aisément déterminée par l'homme du métier à l'aide de ses connaissances générales. Une valeur de référence d'IPDL « seuil » ou de « cut-off » peut être par exemple déterminée comme décrit par Limmathurotsakul et al. (2011, CID, Vol. 52 : 1024-1028).

Selon un aspect préféré de l'invention, les patients présentant un état inflammatoire chronique sont atteints de polyarthrite rhumatoïde.

La présente invention est également relative à un procédé *in vitro* pour déterminer l'efficacité d'un traitement d'un état inflammatoire chronique d'un individu, ledit traitement consistant en l'administration d'une composition comprenant un principe actif inhibiteur de l'IL-17, comprenant les étapes suivantes :
a) déterminer la valeur de IPDL d'un échantillon biologique dudit individu avant le début dudit traitement, par le procédé tel que décrit ci-dessus, et
b) déterminer la valeur de IPDL d'un échantillon biologique dudit individu après le début dudit traitement, par le procédé tel que décrit ci-dessus,
étant entendu que ledit traitement est jugé comme étant efficace lorsque la valeur de IPDL déterminée à l'étape a) est supérieure à la valeur de IPDL déterminée à l'étape b).

Un tel procédé permettant de suivre l'évolution et/ou l'efficacité d'un traitement, correspond à ce que l'on peut appeler un 'test compagnon' qui permet d'adapter ou de changer un traitement, en fonction de la réponse spécifique d'un individu donné à ce traitement, au cours du traitement. Ceci est particulièrement adapté pour des états inflammatoires chroniques, tels qu'observés dans la maladie de polyarthrite rhumatoïde, où les traitements sont de longue durée, et où la réponse du patient à une biothérapie ne peut être prédite à l'avance.

Selon un aspect préféré de l'invention, ledit individu est atteint de polyarthrite rhumatoïde. Selon un autre aspect préféré de l'invention, ledit patient est traité par des anticorps anti-Il-17 monoclonaux. On peut estimer que si le traitement est efficace, la «part fonctionnelle» de l'IL-17 dans le processus d'inflammation devrait être diminuée suite à l'administration d'anticorps neutralisant l'activité de l'IL-17.

Ce procédé *in vitro* pour déterminer l'efficacité d'un traitement d'un état inflammatoire chronique d'un individu par une composition comprenant un principe actif inhibiteur de l'IL-17, peut également comprendre les étapes suivantes :
a) déterminer la valeur de IPDL d'un échantillon biologique dudit individu à un premier instant I1 après le début dudit traitement, par le procédé tel que décrit ci-dessus, et
b) déterminer la valeur de IPDL d'un échantillon biologique dudit individu à un second instant I2, postérieur au premier instant I1, par le procédé tel que décrit ci-dessus,
étant entendu que ledit traitement est jugé comme étant efficace lorsque la valeur de IPDL déterminée à l'étape a) est supérieure à la valeur de IPDL déterminée à l'étape b).

Selon un aspect particulier de l'invention, ledit individu est atteint de polyarthrite rhumatoïde.

L'invention est également relative à un procédé *in vitro* pour déterminer le risque de survenue d'un accident cardio-vasculaire chez un individu affecté d'un état inflammatoire chronique, comprenant les étapes suivantes :
a) déterminer la valeur de IPDL d'un échantillon biologique dudit individu, par le procédé tel que décrit ci-dessus, et
b) comparer la valeur de IPDL obtenue à l'étape a) avec une valeur de référence.

On entend par « accident cardiovasculaire » tout type d'événement néfaste relatif au coeur et/ou aux vaisseaux sanguins. Par exemple, il peut s'agir d'un infarctus du myocarde.

L'invention est également relative à un procédé *in vitro* pour déterminer le risque de destruction osseuse chez un individu affecté d'un état inflammatoire chronique, notamment atteint de polyarthrite rhumatoïde, comprenant les étapes suivantes :
a) déterminer la valeur de IPDL d'un échantillon biologique dudit individu, par le procédé tel que décrit ci-dessus, et
b) comparer la valeur de IPDL obtenue à l'étape a) avec une valeur de référence.

En effet, comme cela est présenté à l'exemple 6 et dans les figures 5A et 5B, il existe un lien entre la présence d'un état inflammatoire chronique chez un individu, et le risque de survenue d'un accident cardio-vasculaire chez ledit individu, et le risque d'observer une destruction osseuse.

On entend par « destruction osseuse » une disparition progressive du tissu osseux due à l'état inflammatoire chronique du patient.

Aux fins de réaliser le procédé ci-dessus, on entend par « valeur de référence », une valeur IPDL mesurée chez des patients affecté d'un état inflammatoire chronique, par exemple une polyarthrite rhumatoïde, et dont la survenue ou non d'un accident cardio-vasculaire est connue, ou un phénomène de destruction osseuse a été observé. En général, ladite valeur de référence est une valeur IPDL moyenne mesurée à partir d'un échantillon biologique d'une pluralité d'individus affectés d'un état inflammatoire chronique, par exemple d'une polyarthrite rhumatoïde, et dont la survenue ou non d'un accident cardio-vasculaire est connue, ou une destruction osseuse est observée, respectivement.

Aux fins de mise en oeuvre des procédés selon l'invention, on peut choisir comme « valeur de référence » une valeur d'IPDL moyenne déterminée ou mesurée chez des patients affectés d'un état inflammatoire chronique, par exemple d'une polyarthrite rhumatoïde, et dont la survenue ou non d'un accident cardio-vasculaire est connue ou une destruction osseuse est observée, respectivement.

Dans certains modes de réalisation du procédé, la valeur de référence est une valeur d'IPDL déterminée chez des patients n'ayant pas subi d'accident cardio-vasculaire, ou ne présentant pas de destruction osseuse. Dans ces modes de réalisation, un patient testé selon le procédé de l'invention sera classé « à risque réduit ou nul d'accident cardio-vasculaire » ou « « à risque réduit ou nul de destruction osseuse » lorsque la valeur d'IPDL mesurée pour ce patient est égale ou inférieure à la valeur IPDL de référence.

Dans certains modes de réalisation du procédé, la valeur de référence est une valeur d'IPDL déterminée chez des patients ayant subi un accident cardio-vasculaire, ou présentant une destruction osseuse. Dans ces modes de réalisation, un patient testé selon le procédé de l'invention sera classé « à risque modéré ou important d'accident cardio-vasculaire » ou « à risque modéré ou important de destruction osseuse » lorsque la valeur d'IPDL mesurée pour ce patient est égale ou supérieure à la valeur IPDL de référence.

Dans encore d'autres modes de réalisation, la valeur de référence est une valeur IPDL dite « seuil » ou de « cut-off », qui est déterminée à partir (i) de valeurs IPDL déterminées chez des patients « à risque réduit ou nul » et (ii) de valeurs IPDL déterminées chez des patients « à risque modéré ou important». Dans ces modes de réalisation, un patient testé selon le procédé de l'invention sera classé « à risque réduit ou nul d'accident cardio-vasculaire » ou « à risque réduit ou nul de destruction osseuse » lorsque la valeur d'IPDL mesurée pour ce patient est inférieure à la valeur IPDL de référence Dans ces modes de réalisation, un patient testé selon le procédé de l'invention sera classé « à risque modéré ou important d'accident cardio-vasculaire » ou « à risque modéré ou important de destruction osseuse » lorsque la valeur d'IPDL mesurée pour ce patient est supérieure à la valeur IPDL de référence.

Une valeur de référence d'IPDL « seuil » ou de « cut-off » peut être aisément déterminée par l'homme du métier à l'aide de ses connaissances générales. Une valeur de référence d'IPDL « seuil » ou de « cut-off » peut être par exemple déterminée comme décrit par Limmathurotsakul et al. (2011, CID, Vol. 52 : 1024-1028).

Selon un aspect particulier de l'invention, ledit individu affecté d'un état inflammatoire chronique est atteint de polyarthrite rhumatoïde, est peut être classé « à risque réduit ou nul d'accident cardio-vasculaire » ou « à risque réduit ou nul de destruction osseuse » en fonction de l'IPDL mesuré dans son échantillon de plasma.

Ainsi, par l'identification de ce nouveau marqueur IPDL, les procédés suivants ont pu être mis au point pour :
- déterminer les chances de réponse d'un patient atteint d'un état inflammatoire chronique, à un traitement comprenant l'administration d'un anticorps anti-IL17;
- déterminer l'efficacité d'un traitement d'un état inflammatoire chronique d'un individu, en particulier lorsque ledit traitement consiste en l'administration d'une composition comprenant un principe actif inhibiteur de l'IL-17 ; et
- déterminer le risque de survenue d'un accident cardio-vasculaire chez un individu affecté d'un état inflammatoire chronique ; et
- déterminer le risque de destruction osseuse chez un individu affecté d'un état inflammatoire chronique.

Il est entendu que pour la mise en oeuvre de tous ces procédés, l'échantillon biologique a été prélevé sur l'individu testé, et qu'il s'agit d'un échantillon de sang total, de plasma, de sérum, de liquide synovial, de liquide céphalo-rachidien, de liquide pleural, ou de liquide péritonéal dudit individu.

Lesdits procédés pourront être réalisés sur tout type de cellules sensibles à l'IL-17, mais seront de préférence caractérisés en ce que les cellules sensibles à l'IL-17 utilisées sont des cultures primaires de cellules, notamment de cellules prélevées sur l'individu dont l'échantillon biologique est testé.

Selon un autre aspect de l'invention, le procédé pour suivre l'efficacité d'un traitement, ou pour déterminer le risque de survenue d'un accident cardio-vasculaire et/ou de destruction osseuse, peut être réalisé sur des cellules sensibles à l'IL-17 immortalisées. L'avantage de ce mode de réalisation est que le même type de cellules peut être utilisé pour tester des échantillons biologiques de patients à différents moments du traitement, et que les résultats seront donc comparables.

La présente invention concerne également un procédé de criblage *in vivo* de composés potentiellement inhibiteurs de l'IL-17 chez un animal non humain, comprenant les étapes suivantes :
a) déterminer la valeur de IPDL d'un échantillon biologique dudit animal avant le début d'un traitement avec ledit composé, par le procédé selon l'invention,
b) administrer à l'animal ledit composé candidat ;
c) déterminer la valeur de IPDL d'un échantillon biologique dudit animal après le début dudit traitement, par le procédé selon l'invention,
étant entendu que ledit composé est jugé comme étant efficace pour inhiber l'IL-17 lorsque la valeur de IPDL déterminée à l'étape a) est supérieure à la valeur de IPDL déterminée à l'étape c).

Selon un aspect de l'invention, ledit procédé de criblage *in vivo* est réalisé sur des animaux de laboratoire, notamment des souris ou des rats. Selon un aspect préféré de l'invention, ces animaux présentent un état inflammatoire chronique. Selon un aspect encore plus préféré de l'invention, ces animaux sont atteints d'une maladie équivalente à la polyarthrite rhumatoïde humaine, ou sont des modèles d'étude de cette maladie.

Différents composés potentiellement inhibiteurs de l'IL-17, ou plutôt de l'action de l'IL-17, pourront ainsi être testés : des anticorps mais également des agents bloquant les récepteurs de l'IL-17 ou neutralisant la voie de signalisation de l'IL-17. Par ce test permettant de déterminer l'IPDL avant et après le début du traitement, on pourra déterminer *in vivo* sur un modèle animal si la contribution fonctionnelle de l'Il-17 dans le processus d'inflammation a été diminuée suite au début du traitement, ou si elle a perduré, indiquant une mauvaise efficacité dudit traitement.

L'étape b) d'administration d'un composé candidat sera réalisée sur une période adéquate pour juger de l'efficacité dudit composé, cette période allant de un jour à plusieurs mois, et étant calquée sur les périodes d'administration habituelles de ce type de composés.

### Kits pour déterminer l'IPDL

La présente demande est également relative à un kit utile pour déterminer le niveau pro-inflammatoire dépendant de l'IL-17 fonctionnelle (IPDL) d'un échantillon biologique prélevé sur un individu, comprenant :
a) un test immunologique de quantification d'un marqueur inflammatoire,
b) un anticorps anti-IL-17, et
c) une lignée immortalisée de cellules synoviales sensibles à l'IL-17.

En particulier, le test immunologique de quantification sera sous la forme d'un test ELISA.

Selon un aspect particulier, ledit marqueur inflammatoire est choisi parmi l'IL-6, l'IL-8, le G-CSF, le GM-CSF et la prostaglandine E2, et de manière tout à fait préférée parmi l'IL-6 et l'IL-8.

Selon un autre aspect particulier, l'anticorps anti-IL-17 est un anticorps monoclonal.

La présente demande décrit également un kit comprenant une lignée immortalisée de cellules sensibles à l'IL-17 choisie parmi une lignée de cellules synoviales, une lignée de cellules épithéliales, une lignée de cellules endothéliales et une lignée de fibroblastes.

Dans un premier aspect, le kit comprend :
- un test ELISA destiné à mesurer le taux de sécrétion d'IL-6 ;
- un anticorps anti-IL17, notamment un anticorps monoclonal anti-IL-17A humaine ; et
- une lignée immortalisée de fibroblastes.

Dans un second aspect, le kit comprend :
- un test ELISA destiné à mesurer le taux de sécrétion d'IL-8 ;
- un anticorps anti-IL17, notamment un anticorps monoclonal anti-IL-17A humaine ; et
- une lignée immortalisée de cellules endothéliales.

En particulier la lignée immortalisée de cellules endothéliales sera issue de la lignée dénommée « HUVEC », constituée de cellules endothéliales de la veine de cordon ombilical humain, ces cellules ayant été modifiées pour créer une lignée cellulaire immortalisée.

Dans un aspect préféré, le kit comprend :
- un test ELISA destiné à mesurer le taux de sécrétion d'IL-6 ;
- un anticorps anti-IL17, notamment un anticorps monoclonal anti-IL-17A humaine ; et
- une lignée immortalisée de cellules synoviales.
Selon ce document, on entend par « cellules synoviales » notamment des cellules de type fibroblastes communément appelées « Fibroblast-Like-Synoviocytes » (FLS).
Par exemple, une lignée cellulaire immortalisée de cellules « Fibroblast-Like-Synoviocytes » (FLS) peut être utilisée dans le cadre de ce document.

### EXEMPLES

### MATÉRIEL ET MÉTHODES

### Isolement et culture de synoviocytes primaires (de type fibroblastes) de patients atteints de polyarthrite rhumatoïde

Des échantillons de synovium ont été obtenus à partir de patients atteints de polyarthrite rhumatoïde ayant subi un remplacement articulaire.

Les tissus ont été hachées et digérés par de la DNase à 0,15 mg / ml (Roche), de l'hyaluronidase à 0,15 mg / ml (Sigma-Aldrich) et de la collagénase de type IA à 1 mg/ml (Sigma-Aldrich) dans du milieu DMEM (Eurobio) pendant 1 heure à 37 ° C. Les cellules ont ensuite été lavées et mises en culture dans du DMEM supplémenté avec 10 % de sérum bovin foetal (Invitrogen), 200 mM de L-glutamine (Lonza), 100U/ml de pénicilline, 100 pg/ml de streptomycine (Lonza), et 5 pg/ml plasmocin (Lonza). Les synoviocytes ont été utilisés au stade « passage quatre à huit ».

### Détermination de l'IPDL - mise au point du test sur IL-17A

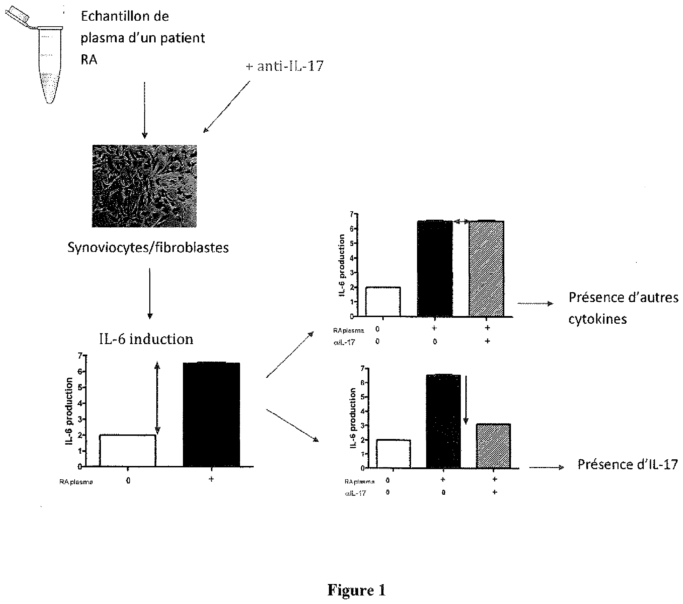

Ce test est schématisé en figure 1. Ce test a été conçu pour déterminer l'IPDL (IL-17 Pro-inflammatory Dependent Level) dans le plasma de patients atteints de polyarthrite rhumatoïde. Des synoviocytes de type fibroblaste (cellules FLS) ont été isolés à partir de patients et ont été cultivées dans des plaques à 96 puits dans du DMEM complet (104 cellules/puits) à 37 ° C / 5 % de CO2 pendant une nuit. Les cellules ont été traitées avec du plasma (heat-decomplemented) provenant de patients dans du DMEM sans sérum pendant 24 h. Pour évaluer l'activité liée à la circulation d'IL-17A, des anticorps anti-IL-17 (R & D system) à différentes concentrations (2,5 , 5, 10 et 20 pg / ml) ont été incubés avec le plasma pendant 1 heure avant l'addition sur les synoviocytes en culture. De l'IL-17 recombinante (R & D Systems) a été utilisée à 50 ng/ml comme contrôle positif. Après 24 h, les surnageants ont été collectés et l'IL-6 a été mesurée par ELISA.

### Détection de l'IL-6 par ELISA

L'IL-6 présente dans les surnageants de culture a été mesurée par ELISA (R & D Systems) selon les instructions du fabricant. Les puits ont été revêtus avec 2.0µg/ml d'anticorps (anti IL-6-humaine) pendant une nuit à température ambiante. Après la phase de saturation avec du tampon PBS contenant 1 % de Bovine Serum Albumine (BSA) pendant 1 heure à température ambiante, les surnageants dilués à 1/250 ou la solution témoin ont été ajoutés dans les puits, et incubés pendant 2 heures. L'anticorps anti-IL-6 humaine est ajouté à 50 ng / ml pendant 2 h. La streptavidine conjuguée à la peroxydase diluée au 1/5 000, est ajoutée dans les puits et le tout est incubé pendant 20 min. La révélation est effectuée par addition d'une solution de substrat (H2O2 + TMB). La densité optique est mesurée avec un lecteur de microplaque à 450 nm.

### Analyse statistique

Toutes les données sont exprimées comme la moyenne ± écart-type. Des corrélations ont été estimées par 2 à queue t - test non paramétrique de Graphpad Prism. Les valeurs de p inférieures à 0,05 ont été considérées comme significatives.

### EXEMPLE 1.

L'IL-17 recombinante humaine induit la production d'IL-6 par les cellules FLS et la présence d'anticorps anti-IL17 à 10 µg/ml inhibe cet effet.

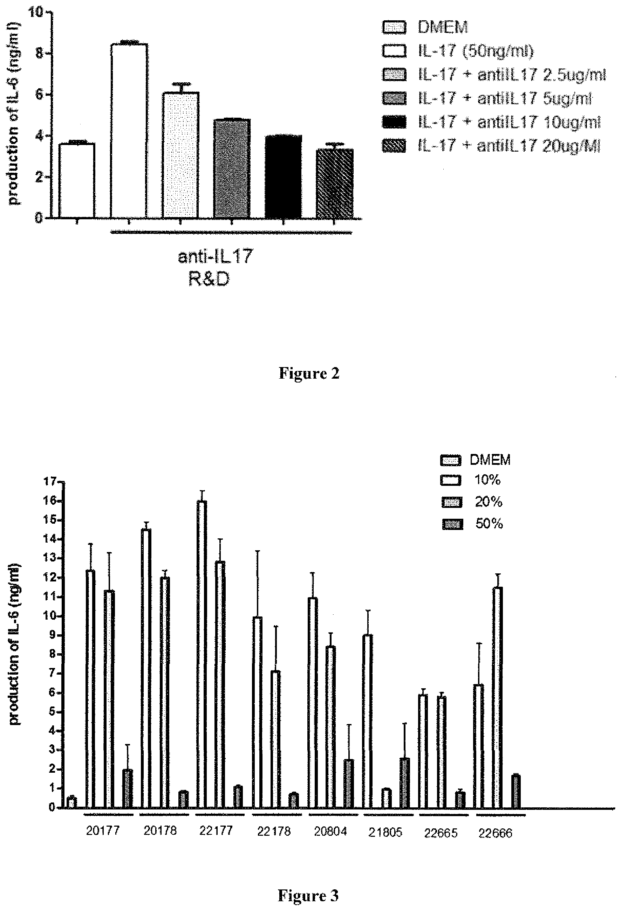

Différentes concentrations d'anticorps anti-IL17 sont testées pour bloquer l'effet de l'IL-17 recombinante humaine à 50 ng/ml sur les cellules FLS ; la production d'IL-6 par ces cellules a été mesurée par ELISA. La stimulation par 50 ng/ml d'IL-17 recombinante pendant 24 heures induit une augmentation de deux fois le niveau dans le surnageant de culture de l'IL-6. De plus, cette expérience permet de valider que les anticorps anti-IL17 inhibent l'effet de l'IL-17 d'une manière dose-dépendante (Figure 2). Les anticorps anti-IL17 utilisés à 10 µg/ml induisent une inhibition de presque 100 % de la production d'IL-6. Ces résultats confirment que l'IL-17 induit une augmentation de l'IL-6 par les cellules FLS issues de patients, et que les anticorps anti-IL17 à 10 µg/ml sont efficaces.

### EXEMPLE 2.

Dans cette seconde étape, plusieurs dilutions des échantillons de plasma des patients ont été testées. Pour cela, les cellules FLS ont été traitées avec différentes concentrations (10, 20 et 50 % en volume) de plasma décomplémenté, et la production d'IL-6 a été mesurée par ELISA. De faibles concentrations de plasma (10% et 20%) induisent une augmentation significative de l'IL-6, tandis que des concentrations élevées de plasma (50 %) n'ont pas d'effet significatif sur la production d'IL-6 (Figure 3). Ce résultat suggère que la faible concentration (10%) de plasma de patients est la concentration favorable pour les expériences ultérieures.

### EXEMPLE 3. L'IPDL est plus élevé chez les patients atteints de polyarthrite rhumatoïde que chez les donneurs sains

Pour étudier l'implication fonctionnelle de l'IL-17 dans la pathogenèse de la polyarthrite rhumatoïde, un test a été mis au point pour déterminer l'IPDL, le niveau pro-inflammatoire dépendant de l'IL-17 fonctionnelle d'un échantillon biologique, en particulier du plasma sanguin. Pour cela, les synoviocytes ou les cellules endothéliales ont été traitées avec 10% de plasma de patients atteints de polyarthrite rhumatoïde et de donneurs sains, en présence ou non d'anticorps anti- IL17 à 10µg/ml Ensuite, le ΔIL-6 (ou ΔIL-8) a été mesuré, correspondant à la production d'IL- 6 avant et après l'addition d'anticorps anti-IL17. Plus le ΔIL-6 est augmenté, plus l'IL-17 présente dans le plasma est 'fonctionnelle'. Cet essai révèle que des patients atteints de polyarthrite rhumatoïde ont un marqueur « ΔIL-6 » ou « IPDL » significativement plus élevé que celui des donneurs sains (figures 4 et 5).

Ainsi, la présence d'anticorps anti-IL17 induit une diminution de la production d'lL-6 des cellules FLS traitées avec 10 % de plasma de patient. Cette observation suggère que l'IL- 17 est présente dans ce plasma, et qu'elle pourrait être impliquée fonctionnellement dans la maladie.

### EXEMPLE 4. Lien entre IPDL et survenue d'événements cardiovasculaires (CVE), et la destruction osseuse, observés chez des patients atteints de polyarthrite rhumatoïde :

Pour explorer l'association IPDL et complications telles que la survenue d'accidents cardio-vasculaires, ou la destruction osseuse, un essai biologique fonctionnel a été conçu, utilisant le plasma de patients et des cellules endothéliales (HUVEC).

Les données cliniques des patients et des donneurs sains sont présentés dans le tableau 1 suivant :

**Tableau 1**

| **Paramètres** | **Donneurs sains (n=30)** | **Patients atteints de polyarthrite rhumatoïde** | | **P value** |
|---|---|---|---|---|
| | | Pas de destruction (n=17) | Destruction osseuse (n=35) | |
| Sexe (M/F) | 10/20 | 7/10 | 8/27 | 0.10 |
| Age (années) | 60.1 +/6 5.5 | 66.2 +/6 10.9 | 68.9+/- 10.9 | 0.38 |
| Durée de la maladie (années) | | 18.7 +/6 8.9 | 22.1 +/- 11.3 | 0.06 |

La production d'IL8 par des cellules HUVEC en présence d'échantillons de plasma, traité ou non avec des anticorps anti-IL-17, a été mesurée par ELISA. Le Δ IL8 (ou IPDL) est plus élevé chez les patients atteints de polyarthrite rhumatoïde avec complication cardio-vasculaire, que chez ceux qui n'ont pas été atteints par un accident cardio-vasculaire (figure 5B). De plus, les patients présentant une destruction osseuse importante ont un IPDL plus élevé comparé à ceux ne présentant pas de destruction osseuse (Figure 5A). Ainsi, un IPDL élevé peut être corrélé avec un risque cardiovasculaire et une destruction osseuse chez un patient atteint de polyarthrite rhumatoïde.

De manière intéressante, aucune différence significative entre les patients atteints de polyarthrite rhumatoïde sans accident cardio-vasculaire ou destruction osseuse et les donneurs sains n'est observée ; cette observation confirme que tous les patients atteints de polyarthrite rhumatoïde ne seront pas sensibles à un traitement à base d'anticorps anti-IL-17, car leur niveau pro-inflammatoire dépendant de l'IL-17 fonctionnelle (IPDL) n'est pas élevé, rendant donc un tel traitement sans objet.

De plus, un fort taux d'IL-17 fonctionnelle (IPDL) a été observé chez des patients se présentant aux urgences avec un infarctus du myocarde. Comme le montre la figure 5C, le taux d'IL-17 bioactive est plus élevé chez les malades que chez les contrôles (p=0.03). Ceci conforte notre hypothèse qu'une valeur élevée du taux d'IL-17 bioactive est un bon indicateur de la survenue d'accidents cardio-vasculaires aigus.

### EXEMPLE 5. Absence de relation entre la quantité d'IL-17 circulante et l'lPDL

Comme montré sur la figure 6, le taux d'IL-17 circulante a été mesuré par deux kits ELISA différents, provenant respectivement de R&D Systems® et de Dendritics®. Une nette différence de résultats est observée avec les deux kits, confirmant le fait que le taux d'IL-17 circulante n'est pas un bon indicateur pour évaluer le niveau pro-inflammatoire chez un patient.

En parallèle, l'IPDL a été mesuré sur les mêmes échantillons de patients - certains patients présentant un fort taux d'IL-17 circulante avec le premier kit, et pas d'IL-17 avec le second kit, notamment le patient 20690, ne présente pas un IPDL significatif, indiquant que l'IL-17 présente était fonctionnelle.

Avec des résultats discordants pour le taux d'IL-17 circulante, l'IPDL apporte une information complémentaire en permettant de distinguer les plasmas contenant ou non de l'IL17 fonctionnelle, et donc les patients susceptibles d'être traités avec des inhibiteurs de l'IL17.

### EXEMPLE 6. Rôle synergique du TNF et de l'IL-17 dans la sécrétion de cytokines/chémokines pro-inflammatoires

La production d'Il-6 et d'IL-8 par des synoviocytes, stimulés par de l'IL-17 recombinante et/ou du TNF recombinant, sont mesurées par des tests ELISA commerciaux. Puis l'IPDL est calculé sur ces mêmes cellules, dans les mêmes conditions.

Comme indiqué dans les graphiques 7A et 7C, la production d'IL-6 et d'IL-8 par les synoviocytes est stimulée à la fois par l'IL-17 et par le TNF. Ainsi, lorsqu'un échantillon biologique complexe est utilisé pour stimuler ces cellules, la part respective de la sécrétion induite par la présence d'Il-17 et par la présence de TNF est indissociable.

Une augmentation de la production d'IL6 ou d'IL8 par les synoviocytes en fonction de la concentration d'IL17, ou de la concentration du TNF, est observée comme prévu. Un effet synergique est obtenu avec une stimulation simultanée de l'IL17 en combinaison avec le TNF (notamment avec les valeurs suivantes : 50 ng/ml d'IL-17 et 2 ng/ml de TNF - voir 7A et 7C).

Les deux graphiques du dessous (7B et 7D) mettent en évidence le fait que l'IPDL mesuré, indiquant la quantité d'IL-17 «libre et biologiquement active » n'est pas directement relatif à la quantité d'IL-17 recombinante ajoutée ; en effet :
- en absence de TNF, l'IPDL est quasi-identique quelle que soit la concentration d'IL-17 ajoutée ; voir les trois premiers points des deux graphiques 7B et 7D;
- en l'absence d'IL-17, l'IPDL est quasiment indétectable, comme attendu ;
- en présence de TNF, la capacité de l'IL-17 à stimuler les synoviocytes dépend de sa concentration : l'IPDL est supérieur à celui observé sans TNF lorsque l'IL-17 est ajoutée à raison de 50 ng/ml, alors que l'IPDL a une valeur équivalente à celui observé en présence de 10 ng/ml d'IL-17 sans TNF.

L'IPDL est une mesure spécifique de l'IL17 «fonctionnelle» car un taux IPDL élevé, indicateur de présence d'IL-17 fonctionnelle, est observé en présence d'une combinaison comprenant une concentration d'IL17 de 50ng/ml, et d'une forte concentration de TNF (de 1 à 2ng/ml), cette situation pouvant être observée de manière physiologique dans un échantillon de plasma.

Par ailleurs, les résultats sont quasi-identiques lorsque le marqueur inflammatoire utilisé est l'IL-6 ou l'IL-8.

### EXEMPLE 7. Comparaison des résultats obtenus sur des cellules endothéliales HUVEC et sur des cellules immortalisées FLS

Dans la mise au point du test fonctionnel, ont d'abord été utilisées des cellules endothéliales HUVEC, pour lesquelles l'IPDL était déterminé grâce à la mesure de l'IL-8 en tant que marqueur inflammatoire.

Des cellules synoviales de type Fibroblast-Like-Synoviocytes (FLS) issues de patients, puis immortalisées, ont ensuite été testées, dans lesquelles l'IPDL a été déterminé grâce à la mesure de l'IL-6 en tant que marqueur inflammatoire, suite à la stimulation.

Les résultats obtenus dans les cellules endothéliales (Figure 8B) sont, dans la majorité des cas, équivalents aux résultats obtenus dans les synoviocytes immortalisés (Figure 8A). Ces dernières montrent en moyenne des taux plus élevés, ce qui traduit une plus grande sensibilité des synoviocytes immortalisés par rapport à des cellules endothéliales HUVEC.

### REFERENCES

Rouvier E, Luciani MF, Mattei MG, Denizot F, Golstein P. CTLA-8, cloned from an activated T cell, bearing AU-rich messenger RNA instability sequences, and homologous to a herpesvirus saimiri gene. J Immunol. 1993 Jun 15;150(12):5445-56.
Kolls JK, Linden A. Interleukin-17 family members and inflammation. Immunity. 2004 Oct;21(4):467-76.
Miossec P, Kolls JK. Targeting IL-17 and TH17 cells in chronic inflammation. Nat Rev DrugDiscov. Oct;11(10):763-76.
Chabaud M, Durand JM, Buchs N, Fossiez F, Page G, Frappart L, Miossec P. Human interleukin-17: A T cell-derived proinflammatory cytokine produced by the rheumatoid synovium. Arthritis Rheum. 1999 May;42(5):963-70.
Turesson C, McClelland RL, Christianson TJ, Matteson EL. Severe extra-articular disease manifestations are associated with an increased risk of first ever cardiovascular events in patients with rheumatoid arthritis. Ann Rheum Dis. 2007 Jan;66(1):70-5.
Fossiez F, Djossou O, Chomarat P, Flores-Romo L, Ait-Yahia S, Maat C, Pin JJ, Garrone P, Garcia E, Saeland S, Blanchard D, Gaillard C, Das Mahapatra B, Rouvier E, Golstein P, Banchereau J, Lebecque S. T cell interleukin-17 induces stromal cells to produce proinflammatory and hematopoietic cytokines. J Exp Med. 1996 Jun 1;183(6):2593-603.
Limmathurotsakul D, Chantratita N, Teerawattanasook N, Piriyagitpaiboon K, Thanwisai A, Wuthiekanun V, Day NP, Cooper B, Peacock SJ. Enzyme-linked immunosorbent assay for the diagnosis of melioidosis: better than we thought. Clin Infect Dis. 2011 Apr 15;52(8):1024-8.

## Revendications

1. Procédé *in vitro* pour déterminer le niveau pro-inflammatoire dépendant de l'IL-17 fonctionnelle (IPDL) d'un échantillon biologique choisi parmi du sang total, du plasma et du sérum, comprenant les étapes suivantes :
a) mesurer le niveau d'un marqueur inflammatoire produit par des cellules sensibles à l'IL-17, incubées en présence dudit échantillon biologique, lesdites cellules sensibles à l'IL-17 étant des cellules endothéliales,
b) mesurer le niveau dudit marqueur inflammatoire produit par lesdites cellules endothéliales incubées en présence dudit échantillon biologique, en présence d'anticorps neutralisant l'activité biologique de l'IL-17, et
c) déterminer la valeur de l'IPDL, qui est la différence entre le niveau dudit marqueur inflammatoire mesuré à l'étape a) et le niveau dudit marqueur inflammatoire mesuré à l'étape b).

2. Procédé selon la revendication 1, ledit marqueur inflammatoire étant choisi parmi l'IL-6, l'IL-8, le G-CSF, le GM-CSF et la prostaglandine E2, et de manière tout à fait préférée parmi l'IL-6 et l'IL-8.

3. Procédé selon l'une des revendications 1 à 2, dans lequel l'échantillon biologique est un échantillon de plasma.

4. Procédé selon l'une des revendications 1 à 3, la mesure du niveau dudit marqueur inflammatoire étant réalisée, respectivement aux étapes a) et b), par un test immunologique, de préférence un test immuno-enzymatique.

5. Procédé selon l'une des revendications 1 à 4, l'anticorps neutralisant l'activité biologique de l'IL-17 étant un anticorps monoclonal.

6. Procédé *in vitro* pour déterminer les chances de réponse d'un patient atteint d'un état inflammatoire chronique, à un traitement comprenant l'administration d'un anticorps anti-IL17, comprenant les étapes suivantes :
a) déterminer la valeur de IPDL d'un échantillon biologique dudit individu, par le procédé selon l'une des revendications 1 à 5, et
b) comparer la valeur de IPDL obtenue à l'étape a) avec une valeur de référence.

7. Procédé *in vitro* pour déterminer l'efficacité d'un traitement d'un état inflammatoire chronique d'un individu, ledit traitement consistant en l'administration d'une composition comprenant un principe actif inhibiteur de l'IL-17, comprenant les étapes suivantes :
a) déterminer la valeur de IPDL d'un échantillon biologique dudit individu avant le début dudit traitement, par le procédé selon l'une des revendications 1 à 5, et
b) déterminer la valeur de IPDL d'un échantillon biologique dudit individu après le début dudit traitement, par le procédé selon l'une des revendications 1 à 5,
étant entendu que ledit traitement est jugé comme étant efficace lorsque la valeur de IPDL déterminée à l'étape a) est supérieure à la valeur de IPDL déterminée à l'étape b).

8. Procédé *in vitro* pour déterminer l'efficacité d'un traitement d'un état inflammatoire chronique d'un individu par une composition comprenant un principe actif inhibiteur de l'IL-17, comprenant les étapes suivantes :
a) déterminer la valeur de IPDL d'un échantillon biologique dudit individu à un premier instant I1 après le début dudit traitement, par le procédé selon l'une des revendications 1 à 5, et
b) déterminer la valeur de IPDL d'un échantillon biologique dudit individu à un second instant I2, postérieur au premier instant I1, par le procédé selon l'une des revendications 1 à 5,
étant entendu que ledit traitement est jugé comme étant efficace lorsque la valeur de IPDL déterminée à l'étape a) est supérieure à la valeur de IPDL déterminée à l'étape b).

9. Procédé *in vitro* pour déterminer le risque de survenue d'un accident cardio-vasculaire chez un individu affecté d'un état inflammatoire chronique, comprenant les étapes suivantes :
a) déterminer la valeur de IPDL d'un échantillon biologique dudit individu, par le procédé selon l'une des revendications 1 à 5, et
b) comparer la valeur de IPDL obtenue à l'étape a) avec une valeur de référence.

10. Procédé *in vitro* pour déterminer le risque de destruction osseuse chez un individu affecté d'un état inflammatoire chronique, comprenant les étapes suivantes :
a) déterminer la valeur de IPDL d'un échantillon biologique dudit individu, par le procédé selon l'une des revendications 1 à 5, et
b) comparer la valeur de IPDL obtenue à l'étape a) avec une valeur de référence.

11. Procédé selon l'une des revendications 6 à 10, **caractérisé en ce que** les cellules sensibles à l'IL-17 sont des cellules prélevées sur l'individu dont l'échantillon biologique est testé.

12. Procédé selon l'une des revendications 1 à 10, **caractérisé en ce que** les cellules sensibles à l'IL-17 sont des cellules immortalisées.

13. Procédé de criblage *in vivo* de composés potentiellement inhibiteurs de l'IL-17 chez un animal non humain, comprenant les étapes suivantes :
a) déterminer la valeur de IPDL d'un échantillon biologique dudit animal avant le début d'un traitement avec ledit composé, par le procédé selon l'une des revendications 1 à 5, et
b) administrer à l'animal ledit composé candidat ;
c) déterminer la valeur de IPDL d'un échantillon biologique dudit animal après le début dudit traitement, par le procédé selon l'une des revendications 1 à 5,
étant entendu que ledit composé est jugé comme étant efficace pour inhiber l'IL-17 lorsque la valeur de IPDL déterminée à l'étape a) est supérieure à la valeur de IPDL déterminée à l'étape c).

14. Kit utile pour déterminer le niveau pro-inflammatoire dépendant de l'IL-17 d'un échantillon biologique choisi parmi du sang total, du plasma et du sérum, prélevé sur un individu, comprenant :
a) un test ELISA destiné à mesurer le taux de sécrétion d'IL-8,
b) un anticorps anti-IL-17, notamment un anticorps monoclonal anti-IL-17A humaine, et
c) une lignée immortalisée de cellules endothéliales.

15. Kit selon la revendication 14, **caractérisée en ce que** ladite lignée immortalisée de cellules endothéliales est issue de la lignée « HUVEC ».

## Patentansprüche

1. *In-vitro*-Verfahren zur Bestimmung des von funktionellem IL-17 abhängigen proinflammatorischen Niveaus (IPDL) einer aus Vollblut, Plasma und Serum ausgewählten biologischen Probe, umfassend die folgenden Schritte:
a) Messen des Niveaus eines Entzündungsmarkers, der von IL-17-sensitiven Zellen produziert wird, die in Gegenwart der biologischen Probe inkubiert werden, wobei die IL-17-sensitiven Zellen Endothelzellen sind,
b) Messen des Niveaus des Entzündungsmarkers, der von den Endothelzellen produziert wird, die in Gegenwart der biologischen Probe inkubiert werden, in Gegenwart von Antikörper, der die biologische Aktivität von IL-17 neutralisiert, und
c) Bestimmen des IPDL-Werts, der die Differenz zwischen dem in Schritt a) gemessenen Niveau des Entzündungsmarkers und dem in Schritt b) gemessenen Niveau des Entzündungsmarkers ist.

2. Verfahren gemäß Anspruch 1, wobei der Entzündungsmarker aus IL-6, IL-8, G-CSF, GM-CSF und Prostaglandin E2 und am meisten bevorzugt aus IL-6 und IL-8 ausgewählt ist.

3. Verfahren gemäß einem der Ansprüche 1 bis 2, wobei die biologische Probe eine Plasmaprobe ist.

4. Verfahren gemäß einem der Ansprüche 1 bis 3, wobei das Messen des Niveaus des Entzündungsmarkers in den Schritten a) bzw. b) durch einen immunologischen Test, vorzugsweise einen immunenzymatischen Test, durchgeführt wird.

5. Verfahren gemäß einem der Ansprüche 1 bis 4, wobei der die biologische Aktivität von IL-17 neutralisierende Antikörper ein monoklonaler Antikörper ist.

6. *In-vitro*-Verfahren zur Bestimmung der Wahrscheinlichkeit einer Ansprechreaktion eines Patienten mit einem chronischen Entzündungszustand auf eine die Verabreichung eines Anti-IL17-Antikörpers umfassende Behandlung, umfassend die folgenden Schritte:
a) Bestimmen des IPDL-Wertes einer biologischen Probe des Individuums durch das Verfahren gemäß einem der Ansprüche 1 bis 5 und
b) Vergleichen des in Schritt a) erhaltenen IPDL-Werts mit einem Referenzwert.

7. *In-vitro*-Verfahren zur Bestimmung der Wirksamkeit einer Behandlung eines chronischen Entzündungszustands eines Individuums, wobei die Behandlung aus der Verabreichung einer Zusammensetzung besteht, die einen IL-17-hemmenden Wirkstoff umfasst, umfassend die folgenden Schritte:
a) Bestimmen des IPDL-Wertes einer biologischen Probe des Individuums vor Beginn der Behandlung durch das Verfahren gemäß einem der Ansprüche 1 bis 5 und
b) Bestimmen des IPDL-Wertes einer biologischen Probe des Individuums nach dem Beginn der Behandlung durch das Verfahren gemäß einem der Ansprüche 1 bis 5,
wobei es sich versteht, dass die Behandlung als wirksam beurteilt wird, wenn der in Schritt a) bestimmte IPDL-Wert größer ist als der in Schritt b) bestimmte IPDL-Wert.

8. *In-vitro-*Verfahren zur Bestimmung der Wirksamkeit einer Behandlung eines chronischen Entzündungszustands eines Individuums mittels einer Zusammensetzung, die einen IL-17-hemmenden Wirkstoff umfasst, umfassend die folgenden Schritte:
a) Bestimmen des IPDL-Wertes einer biologischen Probe des Individuums zu einem ersten Zeitpunkt I1 nach dem Beginn der Behandlung durch das Verfahren gemäß einem der Ansprüche 1 bis 5 und
b) Bestimmen des IPDL-Wertes einer biologischen Probe des Individuums zu einem zweiten Zeitpunkt I2 nach dem ersten Zeitpunkt I1 durch das Verfahren gemäß einem der Ansprüche 1 bis 5,
wobei es sich versteht, dass die Behandlung als wirksam beurteilt wird, wenn der in Schritt a) bestimmte IPDL-Wert größer ist als der in Schritt b) bestimmte IPDL-Wert.

9. *In-vitro*-Verfahren zur Bestimmung des Risikos des Auftretens eines kardiovaskulären Ereignisses bei einem von einem chronischen Entzündungszustand betroffenen Individuum, umfassend die folgenden Schritte:
a) Bestimmen des IPDL-Wertes einer biologischen Probe des Individuums durch das Verfahren gemäß einem der Ansprüche 1 bis 5 und
b) Vergleichen des in Schritt a) erhaltenen IPDL-Werts mit einem Referenzwert.

10. *In-vitro*-Verfahren zur Bestimmung des Risikos der Knochenzerstörung bei einem von einem chronischen Entzündungszustand betroffenen Individuum, umfassend die folgenden Schritte:
a) Bestimmen des IPDL-Wertes einer biologischen Probe des Individuums durch das Verfahren gemäß einem der Ansprüche 1 bis 5 und
b) Vergleichen des in Schritt a) erhaltenen IPDL-Werts mit einem Referenzwert.

11. Verfahren gemäß einem der Ansprüche 6 bis 10, **dadurch gekennzeichnet, dass** die IL-17-sensitiven Zellen Zellen sind, welche aus dem Individuum entnommen wurden, dessen biologische Probe getestet wird.

12. Verfahren gemäß einem der Ansprüche 1 bis 10, **dadurch gekennzeichnet, dass** die IL-17-sensitiven Zellen immortalisierte Zellen sind.

13. Verfahren zum *In-vivo*-Screening von potentiell IL-17-hemmenden Verbindungen in einem nicht-menschlichen Tier, umfassend die folgenden Schritte:
a) Bestimmen des IPDL-Wertes einer biologischen Probe des Tieres vor Beginn einer Behandlung mit der Verbindung durch das Verfahren gemäß einem der Ansprüche 1 bis 5, und
b) Verabreichen der Kandidatenverbindung an das Tier;
c) Bestimmen des IPDL-Wertes einer biologischen Probe des Tieres nach dem Beginn der Behandlung durch das Verfahren gemäß einem der Ansprüche 1 bis 5,
wobei es sich versteht, dass die Verbindung als wirksam zur Hemmung von IL-17 beurteilt wird, wenn der in Schritt a) bestimmte IPDL-Wert größer ist als der in Schritt c) bestimmte IPDL-Wert.

14. Kit, geeignet zur Bestimmung des von IL-17 abhängigen proinflammatorischen Niveaus einer aus Vollblut, Plasma und Serum ausgewählten biologischen Probe, welche aus einem Individuum entnommen wurden, umfassend
a) einen ELISA-Test zum Messen des Sekretionsspiegels von IL-8,
b) einen Anti-IL-17-Antikörper, insbesondere einen monoklonalen humanen Anti-IL-17A-Antikörper, und
c) eine immortalisierte Endothelzelllinie.

15. Kit gemäß Anspruch 14, **dadurch gekennzeichnet, dass** die immortalisierte Endothelzelllinie von der "HUVEC"-Linie abgeleitet ist.

## Claims

1. *In vitro* process for determining the functional IL-17 pro-inflammatory dependent level (IPDL) of a biological sample chosen from whole blood, plasma and serum, comprising the following steps:
a) measuring the level of an inflammatory marker produced by IL-17-sensitive cells incubated in the presence of said biological sample, said IL-17-sensitive cells being endothelial cells,
b) measuring the level of said inflammatory marker produced by said endothelial cells incubated in the presence of said biological sample, in the presence of antibodies which neutralize the biological activity of IL-17, and
c) determining the IPDL value, which is the difference between the level of said inflammatory marker measured in step a) and the level of said inflammatory marker measured in step b).

2. Process according to Claim 1, wherein said inflammatory marker being chosen from IL-6, IL-8, G-CSF, GM-CSF and prostaglandin E2, and entirely preferably from IL-6 and IL-8.

3. Process according to either of Claims 1 and 2, wherein the biological sample is a plasma sample.

4. Process according to one of Claims 1 to 3, the measurement of the level of said inflammatory marker being carried out, respectively in steps a) and b), by means of an immunological test, preferably an immunoenzymatic test.

5. Process according to one of Claims 1 to 4, the antibody which neutralizes the biological activity of IL-17 being a monoclonal antibody.

6. *In vitro* process for determining the chances of a patient, suffering from a chronic inflammatory condition, responding to a treatment comprising the administration of an anti-IL17 antibody, comprising the following steps:
a) determining the IPDL value of a biological sample from said individual, by means of the process according to one of Claims 1 to 5, and
b) comparing the IPDL value obtained in step a) with a reference value.

7. *In vitro* process for determining the efficacy of a treatment for a chronic inflammatory condition of an individual, said treatment consisting of the administration of a composition comprising an IL-17-inhibiting active ingredient, comprising the following steps:
a) determining the IPDL value of a biological sample from said individual before the start of said treatment, by means of the process according to one of Claims 1 to 5, and
b) determining the IPDL value of a biological sample from said individual after the start of said treatment, by means of the process according to one of Claims 1 to 5,
it being understood that said treatment is judged to be effective when the IPDL value determined in step a) is greater than the IPDL value determined in step b).

8. *In vitro* process for determining the efficacy of a treatment for a chronic inflammatory condition of an individual with a composition comprising an IL-17-inhibiting active ingredient, comprising the following steps:
a) determining the IPDL value of a biological sample from said individual at a first instant I1 after the start of said treatment, by means of the process according to one of Claims 1 to 5, and
b) determining the IPDL value of a biological sample from said individual at a second instant 12, after the first instant I1, by means of the process according to one of Claims 1 to 5,
it being understood that said treatment is judged to be effective when the IPDL value determined in step a) is greater than the IPDL value determined in step b).

9. *In vitro* process for determining the risk of occurrence of a cardiovascular event in an individual suffering from a chronic inflammatory condition, comprising the following steps:
a) determining the IPDL value of a biological sample from said individual, by means of the process according to one of Claims 1 to 5, and
b) comparing the IPDL value obtained in step a) with a reference value.

10. *In vitro* process for determining the risk of bone destruction in an individual suffering from a chronic inflammatory condition, comprising the following steps:
a) determining the IPDL value of a biological sample from said individual, by means of the process according to one of Claims 1 to 5, and
b) comparing the IPDL value obtained in step a) with a reference value.

11. Process according to one of Claims 6 to 10, **characterized in that** the IL-17-sensitive cells are cells taken from the individual whose biological sample is tested.

12. Process according to one of Claims 1 to 10, **characterized in that** the IL-17-sensitive cells are immortalized cells.

13. *In vivo* screening process for compounds which are potentially IL-17 inhibitors in a non-human animal, comprising the following steps:
a) determining the IPDL value of a biological sample from said animal before the start of a treatment with said compound, by means of the process according to one of Claims 1 to 5, and
b) administering said candidate compound to the animal;
c) determining the IPDL value of a biological sample from said animal after the start of said treatment, by means of the process according to one of Claims 1 to 5,
it being understood that said compound is judged to be effective for inhibiting IL-17 when the IPDL value determined in step a) is greater than the IPDL value determined in step c).

14. Kit which is of use for determining the IL-17 pro-inflammatory dependent level of a biological sample chosen from whole blood, plasma and serum, taken from an individual, comprising:
a) an ELISA assay intended for measuring the IL-8 secretion level,
b) an anti-IL-17 antibody, in particular an anti-human IL-17A monoclonal antibody, and
c) an immortalized line of endothelial cells.

15. Kit according to Claim 14, **characterized in that** said immortalized line of endothelial cells is derived from the "HUVEC" line.
